# EUROPÄISCHE PATENTSCHRIFT

(11) **EP 3 213 607 B1**
(45) Veröffentlichungstag und Bekanntmachung des Hinweises auf die Patenterteilung: **12.12.2018**
(21) Anmeldenummer: 15790045.7
(22) Anmeldetag: 19.10.2015
(51) Int. Cl.: H05F 3/02

(54) **ELEKTROSTATISCHE ABLEITUNGSEINRICHTUNG**
ELECTROSTATIC DISCHARGER
DISSIPATEUR ÉLECTROSTATIQUE

(30) Priorität: 31.10.2014 DE 102014222265
(43) Veröffentlichungstag der Anmeldung: 06.09.2017
(73) Patentinhaber: Schunk Wien Gesellschaft m.b.H., 1230 Wien (AT)
(72) Erfinder: DENNER, Gerhard, A-1230 Wien (AT); RADINGER, Thomas, A-3004 Weinzierl (AT)
(74) Vertreter: advotec.
(86) Internationale Anmeldenummer: PCT/EP2015/074182
(87) Internationale Veröffentlichungsnummer: WO 2016/066469

(56) Entgegenhaltungen:
- DE-U1-202013 001 243
- US-A1- 2006 228 923

## Beschreibung

Die Erfindung betrifft eine Ableitungseinrichtung zur Ableitung elektrostatischer Ladung von einer Welle mit einer Leiteranordnung mit zumindest zwei an einem Halter angeordneten, aus einer Kohlenstofffaseranordnung gebildeten, biegeelastisch ausgebildeten Leitern, die sich quer zu einer Wellenlängsachse erstrecken, wobei die Leiter über ihre im Halter aufgenommenen Anschlussabschnitte mit einem Erdungsleiter verbindbar sind und jeweils einen Wellenkontaktabschnitt aufweisen, wobei die Wellenkontaktabschnitte eine Kontaktanordnung zur Kontaktierung mit zwei einander gegenüberliegend in einer Wellenkontaktebene W angeordneten Umfangskontaktbereichen eines Wellenumfangs ausbilden, derart, dass die Wellenkontaktabschnitte bei Kontakt zum Wellenumfang tangential zum Wellenumfang angeordnet sind, wobei der Halter zwei auf einer gemeinsamen Schwenkachse angeordnete Halterschenkel aufweist, die jeweils zur Aufnahme eines Anschlussabschnitts eines Leiters dienen und die zur Einstellung eines zwischen den Halterschenkeln ausgebildeten Halterwinkels gegeneinander verschwenkbar und in einer definierten Schwenkstellung arretierbar sind.

Aus der US 7,193,836 ist eine Ableitungseinrichtung zur Ableitung elektrostatischer Ladung von einer Welle bekannt, bei der eine Kohlenstofffaseranordnung aus einer Vielzahl von Filamenten an einem Halter angeordnet ist, der ringförmig ausgebildet und koaxial zur Welle angeordnet ist. Zur Ausbildung eines Berührungskontakts mit der Welle sind Wellenkontaktabschnitte der einzelnen Filamente normal zum Wellenumfang ausgerichtet. Ein Berührungskontakt zwischen den Filamenten und dem Wellenumfang ergibt sich daher lediglich an den axialen Enden der Filamente. Aufgrund der Anordnung der Kohlenstofffaseranordnung an dem ringförmigen Halter ist die bekannte Ableitungseinrichtung lediglich an einer Welle mit definiertem Umfang geeignet. Aus der US2006228923 A1 ist eine Ableitungseinrichtung ferner bekannt, bei der die Kohlenstofffaseranordnung tangential zur Welle angeordnet ist.

Der vorliegenden Erfindung liegt die Aufgabe zugrunde, eine Ableitungseinrichtung vorzuschlagen, die eine sichere drehrichtungsunabhängige Kontaktierung mit einem Wellenumfang ermöglicht und welche darüber hinaus zur Ableitung elektrostatischer Ladung von Wellen mit unterschiedlichen Wellendurchmessern geeignet ist.

Zur Lösung dieser Aufgabe weist die erfindungsgemäße Ableitungseinrichtung die Merkmale des Anspruchs 1 auf.

Erfindungsgemäß ist die Ableitungseinrichtung mit einer biegeelastisch ausgebildeten Leiteranordnung versehen, die zumindest zwei an einem Halter angeordnete, aus einer Kohlenstofffaseranordnung gebildete Leiter aufweist, die sich quer zu einer Wellenlängsachse einer zu kontaktierenden Welle erstrecken. Die Leiter sind über im Halter aufgenommene Anschlussabschnitte mit einem Erdungsleiter verbindbar. Die Leiter weisen jeweils einen Wellenkontaktabschnitt auf, wobei die Wellenkontaktabschnitte eine Kontaktanordnung zur Kontaktierung mit zwei einander gegenüberliegend in einer Wellenkontaktebene angeordneten Umfangskontaktbereichen eines Wellenumfangs ausbilden. Dabei sind die Wellenkontaktabschnitte tangential zum Wellenumfang angeordnet.

Erfindungsgemäß weist der Halter zwei auf eine gemeinsamen Schwenkachse angeordnete, gegeneinander verschwenkbare Halterschenkel auf, die jeweils zur Aufnahme eines Anschlussabschnitts eines Leiters dienen, und die zur Einstellung eines zwischen den Halterschenkeln ausgebildeten Halterwinkels gegeneinander verschwenkbar und in einer definierten Schwenkstellung arretierbar sind.

Unabhängig davon, ob die Leiter der Leiteranordnung einstückig miteinander verbunden sind, also beispielsweise die Leiter jeweils einen Abschnitt der Leiteranordnung ausbilden, oder ob die Leiter unabhängig voneinander als jeweils separat ausgebildete Leiter ausgebildet sind, ermöglicht die Einstellung eines Halterwinkels die Einstellung eines zwischen den zur tangentialen Anlage an den Wellenumfang bestimmten Wellenkontaktabschnitten definierten Kontaktabstands. Insbesondere bei einer geradlinigen Ausbildung der Leiter ist der Kontaktabstand zwischen den Wellenkontaktabschnitten der Leiter durch den Halterwinkel und den Abstand der Schwenkachse des Halters von der Wellenlängsachse vorgegeben. Hieraus resultieren vielfache Anpassungsmöglichkeiten an besondere Installationsbedingungen zur Installation der Ableitungseinrichtung.

Ebenso kann aufgrund der biegeelastischen Ausgestaltung der Leiter durch eine geeignete Einstellung des Halterwinkels bzw. des durch den Abstand der Schwenkachse von der Wellenlängsachse bestimmten Halterabstands die Kontaktkraft vorgegeben werden, mit der die Wellenkontaktabschnitte der Leiter tangential gegen den Wellenumfang anliegen. Eine gesonderte Kontaktkrafteinrichtung, wie beispielsweise eine Federeinrichtung oder dergleichen, die zwischen den Halterschenkeln wirkt, ist demzufolge überflüssig.

Bei einer bevorzugten Ausführungsform sind die Leiter der Leiteranordnung als zwei voneinander unabhängig ausgebildete Leiterstränge ausgebildet, die jeweils über ihre Anschlussabschnitte elektrisch leitend miteinander verbunden sind.

Die elektrisch leitende Verbindung der Anschlussabschnitte miteinander kann insbesondere dann, wenn die Halterschenkel elektrisch nicht leitend, also beispielsweise aus Kunststoff oder Keramik ausgebildet sind, über den Erdungsleiter erfolgen.

Wenn die elektrisch leitende Verbindung der Anschlussabschnitte miteinander über die Halterschenkel erfolgt, kann sogar bei einer voneinander unabhängigen Ausbildung der Leiter deren elektrische Verbindung ohne die Bereitstellung einer extra hierfür vorzusehenden Komponente realisiert werden. Insbesondere kann in diesem Fall der Erdungsleiter ohne direkte Verbindung zu den Leitern an einen der beiden Halterschenkel angeschlossen werden, um die gewünschte elektrische Ableitung zu erzielen.

Zur elektrisch leitenden Ausgestaltung der Halterschenkel ist es besonders vorteilhaft, wenn die Halterschenkel aus einem elektrisch leitenden Material gebildet sind, wobei die Halterschenkel vorzugsweise aus einem Kohlenstoffmaterial ausgebildet sind.

Abgesehen von der guten elektrischen Leitfähigkeit eignet sich in besonderem Maße die Verwendung von Graphit zur Ausgestaltung der Halterschenkel, da Graphit besonders vorteilhafte tribologische Eigenschaften aufweist, die sicherstellen, dass selbst nach einem Einsatz der Ableitungseinrichtung in einer korrosiven Atmosphäre eine Veränderung des Halterwinkels durch eine Relativdrehung der Halterschenkel möglich ist.

Die Schwenkachse ist vorzugsweise durch einen die Halterschenkel miteinander verbindenden Bolzen gebildet, der unabhängig von den Halterschenkeln ausgebildet ist, sodass die Halterschenkel übereinstimmend ausgebildet sein können und vorzugsweise jeweils ein Schwenkauge aufweisen, mit dem die Halterschenkel auf dem Bolzen angeordnet sind.

Wenn in einer bevorzugten Ausführungsform die Halterschenkel aus einem elektrisch leitenden Material und der Bolzen aus einem elektrisch nichtleitenden Material gebildet sind, kann der Bolzen sowohl zur Realisierung der Schwenkachse als auch zur isolierten Anordnung des Halters gegenüber einer Umgebung des Halters, also beispielsweise einem Anbringungsort des Halters, dienen.

Besonders bevorzugt ist es, wenn die Halterschenkel zur Aufnahme der Anschlussabschnitte der Leiter eine Formkavität zur Aufnahme eines die Anschlussabschnitte einbettenden leitfähigen Füllmaterials aufweisen, da durch die einbettende Aufnahme in dem Füllmaterial sowohl eine sichere mechanische Verbindung als auch eine sichere elektrische Kontaktierung der Leiter mit den Halterschenkeln erreicht wird.

Vorzugsweise ist zur gegenseitigen Arretierung der Halterschenkel zwischen den Halterschenkeln eine Rasteinrichtung ausgebildet, die vorzugsweise ineinander greifende, integral mit den Halterschenkeln ausgebildete Rastelemente aufweist, sodass auf eine separate Ausgestaltung einer die Verrastung der Halterschenkel ermöglichenden Komponente verzichtet werden kann. Hierdurch wird insbesondere die Anzahl der zur Ausführung der Ableitungseinrichtung erforderlichen Komponenten oder Bauteile gering gehalten.

Besonders vorteilhaft ist es, wenn die Kohlenstofffaseranordnung der Leiter ein Fasergeflecht aufweist, das mit einer Beschichtung aus einem pyrolytisch abgeschiedenen Kohlenstoff versehen ist, sodass die Beschichtung aus Pyrokohlenstoff nicht nur dazu dient, eine Kontaktoberfläche des Leiters zu verdichten, sondern darüber hinaus auch eine das Fasergeflecht abstützende Hülle ausbildet, die im Zusammenwirken mit dem Fasergeflecht für die gewünschten biegeelastischen Eigenschaften des Leiters sorgt, der eine Biegesteifigkeit aufweist, die insbesondere auch durch die Dicke der Beschichtung beeinflussbar ist.

Als besonders vorteilhaft hat sich die Ausbildung der Beschichtung durch Anwendung des CVI (Chemical Vapor Infiltration)-Verfahrens herausgestellt, da dieses Verfahren nicht nur für die gewünschte Oberflächenbeschichtung, sondern darüber hinaus auch für die Ausbildung von Bindungskräften zwischen den einzelnen Filamenten des Fasergeflechtes sorgt.

Als besonders vorteilhaft erweist sich, wenn das Fasergeflecht als Umhüllung eines sich in Längsrichtung der Leiter erstreckenden unidirektionalen Faserstrangs ausgebildet ist, der im Wesentlichen parallel zueinander verlaufende Filamente aufweist, sodass durch die Faserzwischenräume Kapillaren ausgebildet werden, welche die Ausnutzung von Kapillareffekten zum Abtransport von Fett oder Feuchtigkeit von dem Wellenumfang ermöglichen.

Zur Erhöhung der Biegesteifigkeit des Leiters ist es vorteilhaft, wenn das Fasergeflecht mit einer Harzmatrix versehen ist.

Nachfolgend wird eine vorteilhafte Ausführungsform der Erfindung anhand der Zeichnungen näher erläutert.

Es zeigen:
- **Fig. 1:**: eine isometrische Darstellung einer an einer Welle angeordneten Ableitungseinrichtung mit einem Halter und einer am Halter angeordneten Leiteranordnung;
- **Fig. 2:**: den in **Fig. 1** dargestellten Halter mit zwei um eine Schwenkachse gegeneinander verschwenkbaren Halterschenkeln;
- **Fig. 3:**: einen einzelnen Halterschenkel des in **Fig. 2** dargestellten Halters.

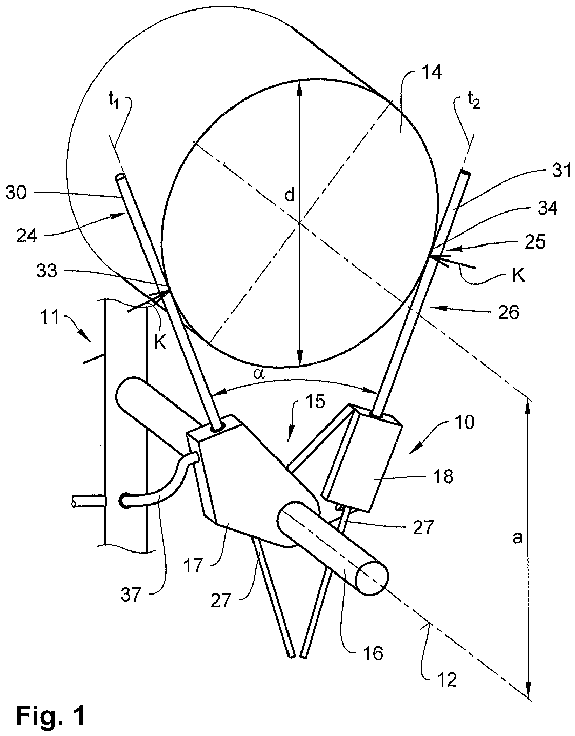

**Fig. 1** zeigt eine Ableitungseinrichtung 10, die in einem Maschinengehäuse 11 angeordnet ist und zur Ableitung einer statischen Aufladung einer im Maschinengehäuse 11 aufgenommenen Welle 14 dient, die beispielsweise als Getriebe- oder Motorwelle ausgebildet sein kann.

Die Ableitungseinrichtung weist einen Halter 15 auf, der zwei auf einem Bolzen 16 gelagerte Halterschenkel 17 und 18 aufweist.

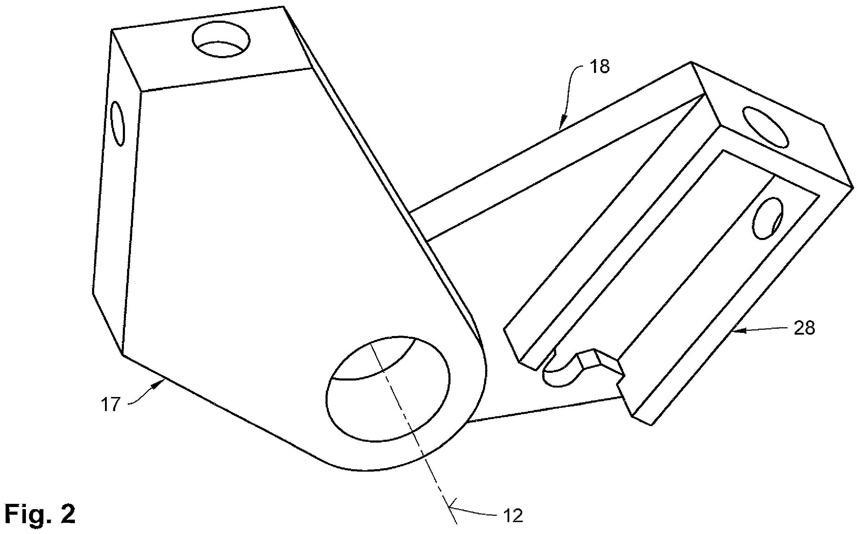

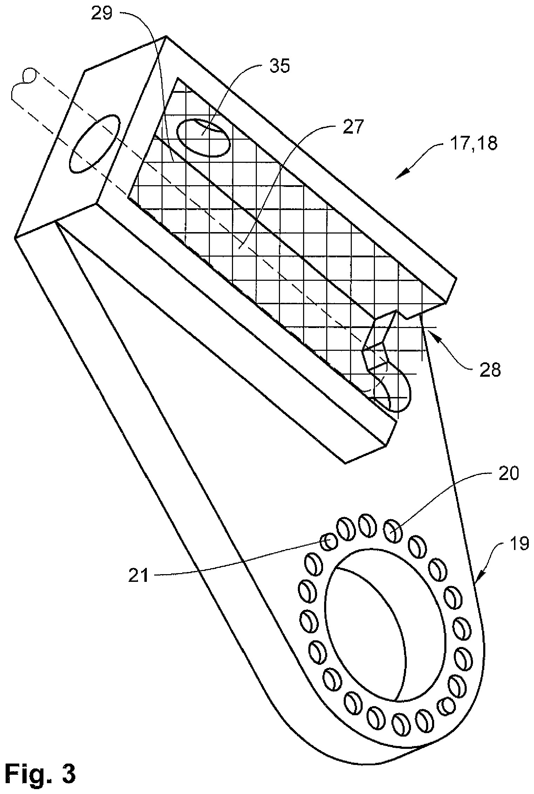

Die den auch in **Fig. 2** dargestellten Halter 15 ausbildenden Halterschenkel 17, 18 sind im vorliegenden Fall identisch ausgebildet und weisen, wie in **Fig. 3** dargestellt, jeweils ein Schwenkauge 19 auf, mit dem die Halterschenkel 17, 18 um eine Schwenkachse 12 auf dem Bolzen 16 verschwenkbar angeordnet sind. Die Halterschenkel 17, 18 weisen jeweils am Umfang des Schwenkauges 19 äquidistant verteilt angeordnete Rastorgane auf, die als Rastöffnungen 20 und als Rastvorsprünge 21 ausgebildet sind. Bei einer Anordnung der Halterschenkel 17, 18 auf dem Bolzen 16, derart, dass Kontaktflächen 22 der Halterschenkel 17, 18, die mit den Rastöffnungen 20 und den Rastvorsprüngen 21 versehen sind, einander gegenüber liegen, kann die beispielhaft in **Fig. 1** dargestellte Schwenkstellung der Halterschenkel 17, 18 mit einem zwischen den Halterschenkeln 17, 18 ausgebildeten Halterwinkel α durch den Eingriff der Rastvorsprünge 21 des einen Halterschenkels 17 in die Rastöffnungen 22 des anderen Halterschenkels 18 fixiert werden. Dabei bilden die Rastvorsprünge 21 des Halterschenkels 17 zusammen mit den Rastöffnungen 22 des Halterschenkels 18 eine Rasteinrichtung 23.

Wie **Fig. 1** zeigt, ist jeweils an den Halterschenkeln 17, 18 ein Leiter 24, 25 einer Leiteranordnung 26 angeordnet, derart, dass die Leiter 24, 25 mit einem Anschlussabschnitt 27 in einer am Halterschenkel 17, 18 jeweils ausgebildeten Formkavität 28, wie in **Fig. 3** dargestellt, aufgenommen ist. Zur mechanisch sicheren Verbindung mit dem Halterschenkel 17, 18 sowie zur elektrischen Kontaktierung mit dem Halterschenkel 17, 18 ist der Anschlussabschnitt 27 im vorliegenden Fall in ein in die Formkavität 28 verfülltes, elektrisch leitfähiges Füllmaterial 29 eingebettet. Alternativ ist es auch möglich, eine elektrisch leitende fixierte Verbindung zwischen dem Anschlussabschnitt 27 und dem Halterschenkel 17, 18 dadurch herzustellen, dass der Anschlussabschnitt 27 über eine hier nicht näher dargestellte durch eine Durchgangsöffnung 35 in die Formkavität 28 hineinragende Klemmschraube oder dergleichen mit dem Halterschenkel 17, 18 kraftschlüssig verbunden ist.

Wie **Fig. 1** zeigt, bilden die im vorliegenden Fall als Kontaktfaserstränge ausgebildeten Leiter 24, 25 mit Wellenkontaktabschnitten 30, 31 entsprechend dem durch die relative Schwenkstellung der Halterschenkel 17, 18 ausgebildeten Halterwinkel α eine V-förmige Leiteranordnung 13, wobei die und liegen an einem Wellenumfang 32 in Umfangskontaktbereichen 33, 34 tangential an, wobei die Wellenkontaktabschnitte 30, 31 aufgrund der biegeelastischen Ausgestaltung der Leiter 17, 18 mit einer Kontaktkraft K in den Umfangskontaktbereichen 33, 34 elastisch federnd anliegen. Dies wird besonders deutlich ersichtlich aus der Darstellung von Berührungstangenten tl und t2, die in **Fig. 1** in den Umfangskontaktbereichen 33, 34 eingezeichnet sind.

Aus der Darstellung in **Fig. 1** wird deutlich, dass die Ableitungseinrichtung 10 grundsätzlich zwei Möglichkeiten zur Beeinflussung der Kontaktkraft K bietet, wobei die eine in einer Veränderung des Halterwinkels α und die andere in der Veränderung des Halterabstands a liegt, wobei der Halterabstand a der Abstand zwischen der Schwenkachse 12 und der Wellenlängsachse 36 der Welle 14 ist. Des Weiteren stellen der Halterwinkel α und der Halterabstand a die möglichen Parameter dar, die eine Anpassung der Ableitungseinrichtung 10 an unterschiedliche Wellendurchmesser d ermöglichen.

Bei dem in **Fig. 1** dargestellten Ausführungsbeispiel erfolgt die elektrischen Ableitung einer Ladung mittels eines an den Halterschenkel 17 angeschlossenen, mit einer isolierenden Ummantelung versehen Erdungsleiters 37, der durch das Maschinengehäuse 11 hindurchgeführt ist.

## Patentansprüche

1. Ableitungseinrichtung (10) zur Ableitung elektrostatischer Ladung von einer Welle (14) mit einer Leiteranordnung (13) mit zumindest zwei an einem Halter (15) angeordneten, aus einer Kohlenstofffaseranordnung gebildeten, biegeelastisch ausgebildeten Leitern (24, 25), die sich quer zu einer Wellenlängsachse (36) erstrecken, wobei die Leiter über im Halter aufgenommene Anschlussabschnitte (27) mit einem Erdungsleiter (37) verbindbar sind und jeweils einen Wellenkontaktabschnitt (30, 31) aufweisen, wobei die Wellenkontaktabschnitte eine Kontaktanordnung zur Kontaktierung mit zwei einander gegenüberliegend in einer Wellenkontaktebene W angeordneten Umfangskontaktbereichen (33, 34) eines Wellenumfangs (32) ausbilden, derart, dass die Wellenkontaktabschnitte bei Kontakt zum Wellenumfang tangential zum Wellenumfang angeordnet sind, **dadurch gekennzeichnet dass** der Halter zwei auf einer gemeinsamen Schwenkachse (12) angeordnete Halterschenkel (17, 18) aufweist, die jeweils zur Aufnahme eines Anschlussabschnitts eines Leiters dienen, und die zur Einstellung eines zwischen den Halterschenkeln ausgebildeten Halterwinkels α gegeneinander verschwenkbar und in einer definierten Schwenkstellung arretierbar sind.

2. Ableitungseinrichtung nach Anspruch 1,
**dadurch gekennzeichnet,**
**dass** die Leiter (17, 18) der Leiteranordnung (13) als zwei voneinander unabhängig ausgebildete Leiterstränge ausgebildet sind, die jeweils über ihre Anschlussabschnitte (27) elektrisch leitend miteinander verbunden sind.

3. Ableitungseinrichtung nach Anspruch 1 oder 2,
**dadurch gekennzeichnet,**
**dass** die Anschlussabschnitte (27) über den Erdungsleiter (37) elektrisch leitend miteinander verbunden sind.

4. Ableitungseinrichtung nach Anspruch 1 oder 2,
**dadurch gekennzeichnet,**
**dass** die Anschlussabschnitte (27) über die Halterschenkel (17, 18) elektrisch leitend miteinander verbunden sind.

5. Ableitungseinrichtung nach Anspruch 4,
**dadurch gekennzeichnet,**
**dass** die Halterschenkel (17, 18) aus einem elektrisch leitenden Material gebildet sind.

6. Ableitungseinrichtung nach Anspruch 5,
**dadurch gekennzeichnet,**
**dass** die Halterschenkel (17, 18) aus einem Kohlenstoffmaterial, insbesondere aus Graphit, gebildet sind.

7. Ableitungseinrichtung nach einem der vorangehenden Ansprüche,
**dadurch gekennzeichnet,**
**dass** die Schwenkachse (12) durch einen die Halterschenkel (17, 18) miteinander verbindenden Bolzen (16) gebildet ist, der unabhängig von den Halterschenkeln ausgebildet ist.

8. Ableitungseinrichtung nach einem der vorangehenden Ansprüche,
**dadurch gekennzeichnet,**
**dass** die Halterschenkel (17, 18) aus einem elektrisch leitenden Material gebildet sind, und der Bolzen (16) aus einem elektrisch nichtleitenden Material gebildet ist.

9. Ableitungseinrichtung nach einem der vorangehenden Ansprüche,
**dadurch gekennzeichnet,**
**dass** die Halterschenkel (17, 18) zur Aufnahme der Anschlussabschnitte (27) der Leiter (24, 25) eine Formkavität (28) zur Aufnahme eines die Anschlussabschnitte einbettenden leitfähigen Füllmaterials (29) aufweisen.

10. Ableitungseinrichtung nach einem der vorangehenden Ansprüche,
**dadurch gekennzeichnet,**
**dass** zur gegenseitigen Arretierung der Halterschenkel (17, 18) zwischen den Halterschenkeln eine Rasteinrichtung (23) ausgebildet ist.

11. Ableitungseinrichtung nach einem der vorangehenden Ansprüche,
**dadurch gekennzeichnet,**
**dass** die Kohlenstofffaseranordnung der Leiter (24, 25) ein Fasergeflecht aufweist, das mit einer Beschichtung aus pyrolytisch abgeschiedenem Kohlenstoff versehen ist.

12. Ableitungseinrichtung nach Anspruch 11,
**dadurch gekennzeichnet,**
**dass** das Fasergeflecht als Umhüllung einer sich in Längsrichtung der Leiter erstreckenden unidirektionalen Faserstrangs ausgebildet ist.

13. Ableitungseinrichtung nach Anspruch 11 oder 12,
**dadurch gekennzeichnet,**
**dass** das Fasergeflecht mit einer Harzmatrix versehen ist.

## Claims

1. Discharging device (10) for discharging electrostatic charges from a shaft (14), comprising a conductor arrangement (13) having at least two bending-elastic conductors (24, 25) arranged on a holder (15) and made of a carbon fiber arrangement and running transverse to a shaft longitudinal axis (36), the conductors being connectable to a grounding conductor (37) via terminal sections (27), which are accommodated in the holder, and each having a shaft contact section (30, 31), the shaft contact sections forming a contact arrangement for making contact with two circumferential contact areas (33, 34) of a shaft circumference (32), the contact areas being arranged opposite each other in a shaft contact plane W, in such a manner that the shaft contact sections are arranged tangentially to the shaft circumference (32) when they are in contact with the shaft circumference,
**characterized in that**
the holder has two holder legs (17, 18) arranged on a common pivot axis (12), each holder leg serving to accommodate a terminal section of a conductor and the holder legs being pivotable against each other in order for a holder angle α formed between the holder legs to be adjusted and the holder legs being lockable in a defined pivoted position.

2. The discharging device according to claim 1,
**characterized in that**
the conductors (17, 18) of the conductor arrangement (13) are realized as two mutually independent conductor strands which are connected to each other in an electrically conductive manner via their terminal sections (27).

3. The discharging device according to claim 1 or 2,
**characterized in that**
the terminal sections (27) are connected to each other in an electrically conductive manner via the grounding conductor (37).

4. The discharging device according to claim 1 or 2,
**characterized in that**
the terminal sections (27) are connected to each other in an electrically conductive manner via the holder legs (17, 18).

5. The discharging device according to claim 4,
**characterized in that**
the holder legs (17, 18) are made of an electrically conductive material.

6. The discharging device according to claim 5,
**characterized in that**
the holder legs (17, 18) are made of a carbon material, in particular of graphite.

7. The discharging device according to any one of the preceding claims,
**characterized in that**
the pivot axis (12) is formed by a bolt (16) connecting the holder legs (17, 18) and being realized independently from the holder legs.

8. The discharging device according to any one of the preceding claims,
**characterized in that**
the holder legs (17, 18) are made of an electrically conductive material and the bolt (16) is made of an electrically non-conductive material.

9. The discharging device according to any one of the preceding claims,
**characterized in that**
for accommodating the terminal sections (27) of the conductors (24, 25), the holder legs (17, 18) have a mold cavity (28) for receiving a conductive filling material (29) embedding the terminal sections.

10. The discharging device according to any one of the preceding claims,
**characterized in that**
a locking means (23) is formed between the holder legs (17, 18) for locking the holder legs relative to each other.

11. The discharging device according to any one of the preceding claims,
**characterized in that**
the carbon-fiber arrangement of the conductors (24, 25) has a fiber network which is provided with a coating made of pyrolytically deposited carbon.

12. The discharging device according to claim 11,
**characterized in that**
the fiber network is realized as an envelope of a unidirectional fiber strand extending in the longitudinal direction of the conductors.

13. The discharging device according to claim 11 or 12,
**characterized in that**
the fiber network is provided with a resin matrix.

## Revendications

1. Dispositif d'évacuation (10) pour l'évacuation de la charge électrostatique d'un arbre rotatif (14), le dispositif d'évacuation comprenant un agencement de conducteurs (13) ayant au moins deux conducteurs (24, 25) élastiques en flexion disposés sur un cadre support (15), réalisés à partir d'un agencement de fibres de carbone et s'étendant perpendiculairement à un axe longitudinal (36) de l'arbre rotatif, lesdits conducteurs pouvant être connectés à un conducteur de terre (37) à travers des sections de connexion (27) qui sont intégrées dans le cadre support et ayant chacun une section de contact (30, 31) avec l'arbre rotatif, lesdites sections de contact avec l'arbre rotatif réalisant un agencement de contact pour la mise en contact avec deux sections de contact périphériques (33, 34) d'une périphérie (32) de l'arbre rotatif disposés de manière opposée l'une vers l'autre dans un plan W de contact d'arbre rotatif, de telle manière que les sections de contact avec l'arbre rotatif sont disposées de manière tangentielle vers la périphérie de l'arbre rotatif lors d'un contact avec la périphérie de l'arbre rotatif,
**caractérisé en ce que**
le cadre support comprend deux branches de cadre support (17, 18) qui sont disposées sur un axe de pivotement (12) commun et dont chacune sert à intégrer une section de connexion d'un conducteur et qui peuvent être pivotées l'une vers l'autre pour le réglage d'un angle de cadre support α réalisé entre les branches de cadre support et qui peuvent être arrêtées dans une position pivotée définie.

2. Dispositif d'évacuation selon la revendication 1,
**caractérisé en ce que**
les conducteurs (17, 18) de l'agencement de conducteurs (13) sont réalisés comme deux cordes conductrices mutuellement indépendantes qui sont connectées l'une à l'autre de manière conductrice de l'électricité à travers leurs sections de connexion (27).

3. Dispositif d'évacuation selon la revendication 1 ou 2,
**caractérisé en ce que**
les sections de connexion (27) sont connectées l'une à l'autre de manière conductrice de l'électricité à travers le conducteur de terre (37).

4. Dispositif d'évacuation selon la revendication 1 ou 2,
**caractérisé en ce que**
les sections de connexion (27) sont connectées l'une à l'autre de manière conductrice de l'électricité à travers les branches de cadre support (17, 18).

5. Dispositif d'évacuation selon la revendication 4,
**caractérisé en ce que**
les branches de cadre support (17, 18) sont réalisées à partir d'un matériau conducteur de l'électricité.

6. Dispositif d'évacuation selon la revendication 5,
**caractérisé en ce que**
les branches de cadre support (17, 18) sont réalisés à partir d'un matériau en carbone, particulièrement de graphite.

7. Dispositif d'évacuation selon l'une quelconque des revendications précédentes,
**caractérisé en ce que**
l'axe de pivotement (12) est réalisé par une cheville (16) connectant les branches de cadre support (17, 18) l'une à l'autre et étant réalisée de manière indépendante des branches de cadre support.

8. Dispositif d'évacuation selon l'une quelconque des revendications précédentes,
**caractérisé en ce que**
les branches de support (17, 18) sont réalisées à partir d'un matériau conducteur de l'électricité et la cheville (16) est réalisée à partir d'un matériau non conducteur de l'électricité.

9. Dispositif d'évacuation selon l'une quelconque des revendications précédentes,
**caractérisé en ce que**
pour l'intégration des sections de connexion (27) des conducteurs (24, 25), les branches de support (17, 18) comprennent une cavité de moule (28) pour l'intégration d'une masse de remplissage (29) conductrice encastrant les sections de connexion.

10. Dispositif d'évacuation selon l'une quelconque des revendications précédentes,
**caractérisé en ce qu'**
un dispositif d'encliquetage (23) pour l'arrêt réciproque des branches de support (17, 18) est réalisé entre les branches de support.

11. Dispositif d'évacuation selon l'une quelconque des revendications précédentes,
**caractérisé en ce que**
l'agencement de fibres de carbone des conducteurs (24, 25) comprend un treillis en fibre qui est fourni d'un revêtement de carbone déposé par pyrolyse.

12. Dispositif selon la revendication 11,
**caractérisé en ce que**
le treillis en fibre est réalisé comme enveloppe d'un faisceau de fibres unidirectionnel s'étendant dans la direction longitudinale des conducteurs.

13. Dispositif d'évacuation selon la revendication 11 ou 12,
**caractérisé en ce que**
le treillis en fibre est fourni d'une matrice de résine.
